# EUROPEAN PATENT SPECIFICATION

(11) **EP 4 259 652 B1**
(45) Date of publication and mention of the grant of the patent: **19.02.2025**
(21) Application number: 21839072.2
(22) Date of filing: 10.12.2021
(51) Int. Cl.: C07K 14/78, C07K 16/18, G01N 33/68, G01N 33/576

(54) **ASSAY FOR DETECTING COLLAGEN XVIII BIOMARKERS**
TEST ZUM NACHWEIS VON KOLLAGEN-XVIII-BIOMARKERN
TEST DE DÉTECTION DES BIOMARQUEURS DU COLLAGÈNE XVIII

(30) Priority: 11.12.2020 GB 202019578
(43) Date of publication of application: 18.10.2023
(73) Proprietor: Nordic Bioscience A/S, 2730 Herlev (DK)
(72) Inventor: VILLESEN, Ida, Falk, 2730 Herlev (DK); LEEMING, Diana, Julie, 2730 Herlev (DK); LANGHOLM, Lasse, 2730 Herlev (DK); FISKER, Mette, Juul, 2730 Herlev (DK); KARSDAL, Morten, Asser, 2730 Herlev (DK)
(74) Representative: Beck Greener LLP
(86) International application number: PCT/EP2021/085308
(87) International publication number: WO 2022/123058

(56) References cited:
- WO-A1-2014/170312
- JANNA SAARELA ET AL: "The Short and Long Forms of Type XVIII Collagen Show Clear Tissue Specificities in Their Expression and Location in Basement Membrane Zones in Humans", THE AMERICAN JOURNAL OF PATHOLOGY, vol. 153, no. 2, 1 August 1998 (1998-08-01), US, pages 611 - 626, XP055472990, ISSN: 0002-9440, DOI: 10.1016/S0002-9440(10)65603-9
- HELJASVAARA RITVA ET AL: "Collagen XVIII in tissue homeostasis and dysregulation - Lessons learned from model organisms and human patients", MATRIX BIOLOGY, ELSEVIER, NL, vol. 57, 13 October 2016 (2016-10-13), pages 55 - 75, XP029931057, ISSN: 0945-053X, DOI: 10.1016/J.MATBIO.2016.10.002

## Description

### Field of the Invention

The present invention relates to an immunoassay for detecting the long and/or intermediate isoforms of type XVIII and/or quantitating the amount of said isoforms in a patient sample. The immunoassay is used for detecting or monitoring liver fibrosis in a patient or for assessing, in a patient with liver fibrosis, the likelihood of the patient responding to treatment with an antifibrotic drug.

### Background

Chronic liver disease (CLD) is a major cause of mortality and morbidity, accounting for over 2 million deaths per year worldwide. Approximately 1 million of these CLD related deaths is due to viral hepatitis, of which hepatitis C is a leading cause¹. Common amongst CLD aetiologies is the development of liver fibrosis with the eventual endpoint being liver cirrhosis. Liver fibrosis is the result of a protracted wound healing response and is characterised by the accumulation of extracellular matrix (ECM), such that the ECM composition of the liver is qualitatively and quantitatively different from that of a healthy one. Recent reports have indicated that the ECM composition of the fibrotic liver is a key determinant of patient survival, which suggests the value of tissue profiling patients. Fibrotic lesions are primarily composed of collagens, which can be broadly divided into two groups; interstitial matrix and basement membrane. The basement membrane regulates cellular function and is a reservoir of growth factors. The accumulation of basement membrane matrix is associated with a reparative tissue response. Conversely, the accumulation of interstitial matrix is associated with more severe and progressive disease.

The multiplexin type XVIII collagen is unique amongst the collagen family. Type XVIII collagen is a heparan sulphate proteoglycan located in the basement membrane, where it interacts with laminin, perlecan, nidogen, fibulins, type IV and VI collagens, playing an essential role in the flexibility of the matrix milieu^{2,3}. Three isoforms exist with differences in their respective distribution; the short isoform is found mostly in the vasculature and skeletal muscle, whereas the intermediate and long isoforms are found predominantly in the liver where it is produced by hepatocytes^{4,5}. All three isoforms carry a cell attachment site in the triple-helical region and the C-terminal fragments endostatin, known to have antiangiogenic and tumour growth inhibitory properties⁶. Nevertheless, the three isoforms do differ in their primary structure, with the most significant difference being the frizzled domain. Located in the N-terminal end on the long isoform, the frizzled (Fz) domain is a transmembrane receptor serving as a binding site for Wnt molecules with the ability to regulate cell fate, proliferation and migration. The Fz domain has been shown to regulate human cancer-cell proliferation and cell-cycle progression in both *in vitro* and *in vivo* studies^{7,8}. In addition, Fz was found to determine adipocyte maturation and abundance in mice⁹. Hence the long isoform of type XVIII collagen carries a broad range of functions potentially affecting liver pathology and hepatocyte function.

The severity of liver fibrosis is typically assessed through either the use of a liver biopsy or transient elastrography. However, liver biopsy is an inherently flawed modality that is associated with high economic and labour costs; and transient elastography, although an attractive non-invasive alternative, is not widely available and does not provide information regarding the dynamics of liver fibrosis.

Consequently, there is a need in the art for novel methods of assessing the presence and/or severity of liver fibrosis in a patient. In particular, serological biomarkers that may be used clinically to identify individuals likely to progress in disease or respond to anti-fibrotic therapy would be of great utility.

### Summary

The invention is defined in the appended claims.

The applicant has now developed an enzyme-linked immunosorbent assay (ELISA) biomarker that can accurately quantify the liver specific isoforms of type XVIII collagen, the long and intermediate isoforms, and has evaluated its clinical value in patients with mild to severe liver fibrosis.

Accordingly, the present invention provides a method of immunoassay comprising: (i) contacting a biofluid sample from a patient with a monoclonal antibody that specifically binds to the long and/or intermediate isoforms of type XVIII and that does not specifically bind to the short isoform of type XVIII; and (ii) detecting and determining the amount of binding between said monoclonal antibody and peptides in the sample.

As used herein the term "amount of binding" refers to the quantification of binding between the monoclonal antibody and peptides in the patient sample. Said quantification may for example be determined by comparing the measured values of binding in the patient sample against a calibration curve produced using measured values of binding in standard samples containing known concentrations of a peptide to which the antibody specifically binds, in order to thereby determine the quantity of peptide to which the antibody specifically binds in the patient sample. In the Examples set out below, an ELISA method is used in which spectrophotometric analysis is used to measure the amount of binding both in the patient samples and when producing the calibration curve. However, any suitable analytical method can be used.

In a preferred embodiment, the patient sample is from a patient with liver fibrosis and the method is for assessing the likelihood of the patient responding to treatment with an antifibrotic drug. The method may further comprise: (iii) correlating the amount of binding of said monoclonal antibody as determined in step (ii) with values associated with responders to treatment and/or to values associated with non-responders and/or a predetermined cut-off value. The patient sample may be from a patient with chronic liver disease. The patient sample may, for example, be from a patient with viral hepatitis, alcoholic liver disease, or non-alcoholic fatty liver disease.

In this context, the term "values associated with responders to treatment" means standardised quantities of binding determined by the method described supra for samples from patients known to have subsequently responded to treatment with an antifibrotic drug, e.g. in whom there was a decrease in liver fibrosis following treatment with the antifibrotic drug (as for example as determined by a decrease in one or more Ishak scores over the course of treatment); and the term "values associated with non-responders" means standardised quantities of binding determined by the method described supra for samples from patients known to have subsequently not responded to treatment with an antifibrotic drug, e.g. in whom there was an increase in liver fibrosis following treatment with the antifibrotic drug.

Also in this context, the term "predetermined cut-off value" means an amount of binding that is determined statistically to distinguish between patients that are less likely to respond to treatment with an antifibrotic drug and patients that are more likely to respond to treatment with an antifibrotic drug. For example, the predetermined cut-off value may be calculated from a previous analysis of levels on binding in samples from liver fibrosis patients who were subsequently treated with an antifibrotic drug or a placebo, wherein in patients with an amount of binding below the cut-off value there was no statistically significant difference between the placebo and antifibrotic drug treatment groups in terms of the number of patients in whom there was a decrease in liver fibrosis or the number of patients in whom there was an increase in liver fibrosis, and/or wherein in patients with a value above the cut-off value there was a statistically significant difference between the placebo and antifibrotic drug treatment groups in terms of the number of patients in whom there was a decrease in liver fibrosis and/or the number of patients in whom there was an increase in liver fibrosis.

In another embodiment, the method is for monitoring liver fibrosis in a patient, the method further comprising: (iii) correlating said amount of binding of said monoclonal antibody as determined in step (ii) with values obtained from said patient at a previous time point. The patient may be a patient with chronic liver disease. The patient may, for example, be a patient with viral hepatitis, alcoholic liver disease, or non-alcoholic fatty liver disease.

Accordingly, in this embodiment the method may comprise quantifying the amount of binding between the monoclonal antibody and peptides in at least two samples obtained from a patient at a first time point and at at least one subsequent time point.

The patient may be a patient undergoing treatment with an antifibrotic drug. In this case, the method may further be for evaluating the efficacy of a drug for treating liver fibrosis, whereby the method comprises quantifying the amount of binding between the monoclonal antibody and peptides in at least two samples obtained from a patient at a first time point and at at least one subsequent time point during a period of administration of the drug to the patient.

In another embodiment, the method is for detecting liver fibrosis in a patient. The method may further comprises: (iii) correlating said amount of binding of said monoclonal antibody as determined in step (ii) with values associated with normal healthy subjects and/or values associated with known disease severity and/or a predetermined cut-off value. In certain embodiments, the method may be for detecting chronic liver disease in a patient.

In this context, the term "values associated with normal healthy subjects" means standardised quantities of binding determined by the method described supra for samples from subjects considered to be healthy, i.e. without disease (and more specifically without liver fibrosis); and the term "values associated with known disease severity" means standardised quantities of binding determined by the method described supra for samples from patients known to have liver fibrosis of a known severity.

Also in this context, the term "predetermined cut-off value" means an amount of binding that is determined statistically to be indicative of a high likelihood of liver fibrosis in a patient, in that a measured value of binding in a patient sample that is at or above the statistical cut-off value corresponds to at least a 70% probability, preferably at least an 80% probability, preferably at least an 85% probability, more preferably at least a 90% probability, and most preferably at least a 95% probability of the presence or likelihood of liver fibrosis.

In preferred embodiments, the sample is a biofluid. The biofluid may be, but is not limited to, blood, serum, plasma, urine or a supernatant from cell or tissue cultures. Preferably the biofluid is blood, serum or plasma.

The immunoassay may be, but is not limited to, a competition assay or a sandwich assay. The immunoassay may, for example, be a radioimmunoassay or an enzyme-linked immunosorbent assay (ELISA). Such assays are techniques known to the person skilled in the art.

The monoclonal antibody specifically binds to the amino acid sequence NLLNLN (SEQ ID NO. 1). Said amino acid sequence is present at the N-terminus of the long and intermediate isoforms of type XVIII (and is absent from the short isoform of type XVIII), and accordingly said monoclonal antibodies specifically bind to the N-terminus of the long and intermediate isoforms of type XVIII.

In certain embodiments, the monoclonal antibody is a monoclonal antibody raised against a synthetic peptide having the amino acid sequence NLLNLNKDKE (SEQ ID NO. 2).

As used herein the term "monoclonal antibody" refers to both whole antibodies and to fragments thereof that retain the binding specificity of the whole antibody, such as for example a Fab fragment, F(ab')2 fragment, single chain Fv fragment, or other such fragments known to those skilled in the art. As is well known, whole antibodies typically have a "Y-shaped" structure of two identical pairs of polypeptide chains, each pair made up of one "light" and one "heavy" chain. The N-terminal regions of each light chain and heavy chain contain the variable region, while the C-terminal portions of each of the heavy and light chains make up the constant region. The variable region comprises three complementarity determining regions (CDRs), which are primarily responsible for antigen recognition. The constant region allows the antibody to recruit cells and molecules of the immune system. Antibody fragments retaining binding specificity comprise at least the CDRs and sufficient parts of the rest of the variable region to retain said binding specificity.

In the methods of the present invention, a monoclonal antibody comprising any constant region known in the art can be used. Human constant light chains are classified as kappa and lambda light chains. Heavy constant chains are classified as mu, delta, gamma, alpha, or epsilon, and define the antibody's isotype as IgM, IgD, IgG, IgA, and IgE, respectively. The IgG isotype has several subclasses, including, but not limited to IgGl, IgG2, IgG3, and IgG4. The monoclonal antibody may preferably be of the IgG isotype, including any one of IgGl, IgG2, IgG3 or IgG4.

The CDR of an antibody can be determined using methods known in the art such as that described by Kabat et al. Antibodies can be generated from B cell clones as described in the examples. The isotype of the antibody can be determined by ELISA specific for human IgM, IgG or IgA isotype, or human IgG1, IgG2, IgG3 or IgG4 subclasses. The amino acid sequence of the antibodies generated can be determined using standard techniques. For example, RNA can be isolated from the cells, and used to generate cDNA by reverse transcription. The cDNA is then subjected to PCR using primers which amplify the heavy and light chains of the antibody. For example primers specific for the leader sequence for all VH (variable heavy chain) sequences can be used together with primers that bind to a sequence located in the constant region of the isotype which has been previously determined. The light chain can be amplified using primers which bind to the 3' end of the Kappa or Lamda chain together with primers which anneal to the V kappa or V lambda leader sequence. The full length heavy and light chains can be generated and sequenced.

The monoclonal antibody or fragment thereof may preferably comprise one or more complementarity-determining regions (CDRs) selected from:

| | |
|---|---|
| CDR-H1: | DYWIH (SEQ ID NO. 3) |
| CDR-H2: | EINPSDGRSNYNEKFKT (SEQ ID NO. 4) |
| CDR-H3: | DLGFAD (SEQ ID NO. 5) |
| CDR-L1: | RSSQSIVYSNGNTYLQ (SEQ ID NO. 6) |
| CDR-L2: | KVSNRFS (SEQ ID NO. 7) |
| CDR-L3: | FQGSHVPFT (SEQ ID NO. 8) |

Preferably the antibody or fragment thereof comprises at least 2,3,4,5 or 6 of the above listed CDR sequences.

Preferably the monoclonal antibody or fragment thereof has a light chain variable region comprising the CDR sequences

| | |
|---|---|
| CDR-L1: | RSSQSIVYSNGNTYLQ (SEQ ID NO. 6) |
| CDR-L2: | KVSNRFS (SEQ ID NO. 7) and |
| CDR-L3: | FQGSHVPFT (SEQ ID NO. 8) |

Preferably the monoclonal antibody or fragment thereof has a light chain that comprises framework sequences between the CDRs, wherein said framework sequences are substantially identical or substantially similar to the framework sequences between the CDRs in the light chain sequence below (in which the CDRs are shown in bold and underlined, and the framework sequences are shown in italics)

RSSQSIVYSNGNTYLQWYLQKPGQSPQLLIYKVSNRFSGVPDRFSGSGSGTDFTLKISRVEA
EDVGVYYCFQGSHVPFT (SEQ ID NO. 9)

Preferably the monoclonal antibody or fragment thereof has a heavy chain variable region comprising the CDR sequences

| | |
|---|---|
| CDR-H1: | DYWIH (SEQ ID NO. 3) |
| CDR-H2: | EINPSDGRSNYNEKFKT (SEQ ID NO. 4) and |
| CDR-H3: | DLGFAD (SEQ ID NO. 5) |

Preferably the monoclonal antibody or fragment thereof has a heavy chain that comprises framework sequences between the CDRs, wherein said framework sequences are substantially identical or substantially similar to the framework sequences between the CDRs in the heavy chain sequence below (in which the CDRs are shown in bold and underlined, and the framework sequences are shown in italics)

DYWIHWVKQRPGQGLEWIGEINPSDGRSNYNEKFKTKATLTVDRSSSTAYMHLSSLTSEDSA
VFYCARDLGFAD (SEQ ID NO. 10)

As used herein, the framework amino acid sequences between the CDRs of an antibody are substantially identical or substantially similar to the framework amino acid sequences between the CDRs of another antibody if they have at least 70%, 80%, 90% or at least 95% similarity or identity. The similar or identical amino acids may be contiguous or non-contiguous.

The framework sequences may contain one or more amino acid substitutions, insertions and/or deletions. Amino acid substitutions may be conservative, by which it is meant the substituted amino acid has similar chemical properties to the original amino acid. A skilled person would understand which amino acids share similar chemical properties. For example, the following groups of amino acids share similar chemical properties such as size, charge and polarity: Group 1 Ala, Ser, Thr, Pro, Gly; Group 2 Asp, Asn, Glu, Gln; Group 3 His, Arg, Lys; Group 4 Met, Leu, Ile, Val, Cys; Group 5 Phe Thy Trp.

A program such as the CLUSTAL program to can be used to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention. Identity or similarity is preferably calculated over the entire length of the framework sequences.

In certain preferred embodiments, the monoclonal antibody or fragment thereof may comprise the light chain variable region sequence: 
DILMTQTPLSLPVGLGDQASISCRSSQSIVYSNGNTYLQWYLQKPGQSPQLLIYKVSNRFSG
VPDRFSGSGSGTDFTLKISRVEAEDVGVYYCFQGSHVPFTFGSGTKLEIK (SEQ ID NO.
11)
 and/or the heavy chain variable region sequence: 
QVQLQQPGAELVKPGASVKLSCKASGYTFIDYWIHWVKQRPGQGLEWIGEINPSDGRSNYNE
KFKTKATLTVDRSSSTAYMHLSSLTSEDSAVFYCARDLGFADWGPGTTVTVSS (SEQ ID
NO. 12)
 (CDRs bold and underlined; Framework sequences in italics) Provided herein is a monoclonal antibody that specifically binds to the long and/or intermediate isoforms of type XVIII and that does not specifically bind to the short isoform of type XVIII.

The monoclonal antibody specifically binds to the amino acid sequence NLLNLN (SEQ ID NO. 1) .

The monoclonal antibody may be a monoclonal antibody raised against a synthetic peptide having the amino acid sequence NLLNLNKDKE (SEQ ID NO. 2).

Further preferred embodiments and features of the monoclonal antibody provided herein will be apparent from the above discussion of the preferred monoclonal antibodies for use in the method of the invention. In particular, the monoclonal antibody may preferably comprise one or more complementarity-determining regions (CDRs), framework sequences and/or variable region sequences as described above.

Also provided herein is an immunoassay kit comprising: (a) a monoclonal antibody that specifically binds to the long and/or intermediate isoforms of type XVIII and that does not specifically bind to the short isoform of type XVIII; and (b) at least one of:
- a streptavidin coated well plate;
- a biotinylated peptide to which the monoclonal antibody specifically binds;
- a secondary antibody for use in a sandwich immunoassay;
- a calibrator peptide to which the monoclonal antibody specifically binds;
- an antibody biotinylation kit;
- an antibody HRP labeling kit;
- an antibody radiolabeling kit; and
- an assay visualization kit.

In a preferred embodiment, the monoclonal antibody specifically binds to the amino acid sequence NLLNLN (SEQ ID NO. 1). The monoclonal antibody may be raised against a synthetic peptide having the amino acid sequence NLLNLNKDKE (SEQ ID NO. 2). In particular, the monoclonal antibody is preferably a monoclonal antibody as described above.

In certain embodiments the calibrator protein may be a peptide comprising the amino acid sequence NLLNLNKDKE (SEQ ID NO. 2). In certain embodiments the biotinylated peptide may comprise NLLNLNKDKE-L-Biotin (SEQ ID NO. 13), wherein L is an optional linker.

### Figures

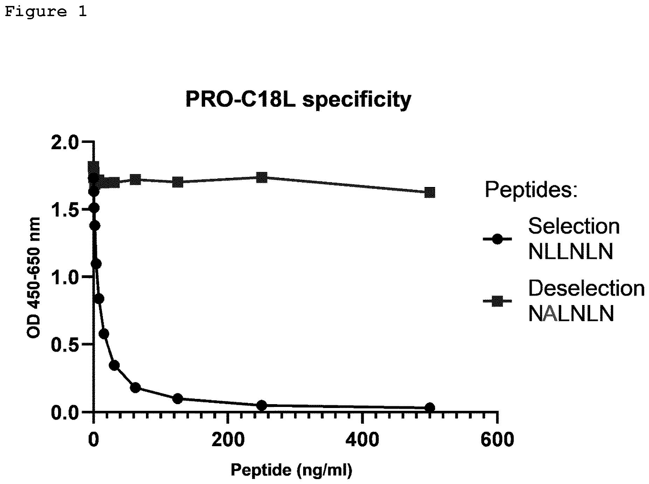

**Figure 1****:** Specificity test of the PRO-C18L assay. The reactivity of the PRO-C18L antibody was tested against the PRO-C18L peptide (standard peptide) and a deselection peptide with one amino acid altered from the PRO-C18L N-terminus sequence. Data is shown as an inhibition curve with optical density (OD) on the y-axis and peptide content (ng/ml) on the x-axis.

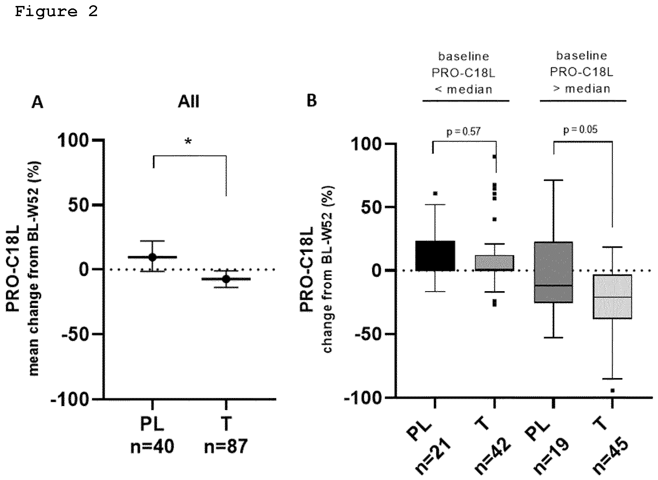

**Figure 2****:** Percentage change in PRO-C18L in placebo (PL) or farglitazar treated (T) patients from baseline (BL) to week 52 (W52) in all patients (A) and in all patients separated into below or above median values of PRO-C18L at baseline. Graphs are shown as box plots with Tukey whiskers. Significance of p<0.05 is indicated with * (A) or exact values (B).

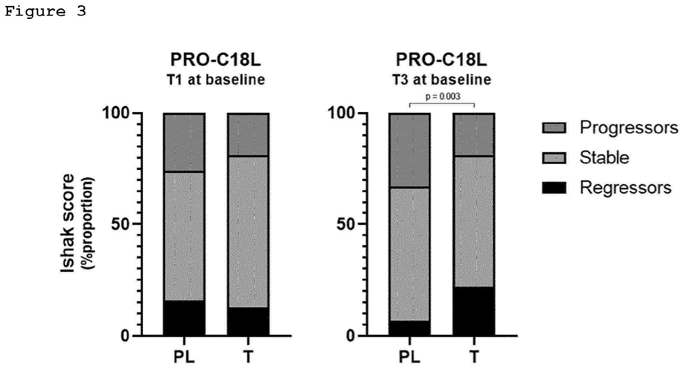

**Figure 3****:** Proportions of regressors, stable or progressors in patient groups divided according to tertiles of baseline PRO-C18L and whether treated with placebo (PL) or farglitazar (T). Patients were characterized as regressors, stable or progressors based on one or more decrease or increase, respectively, in Ishak scoring at week 52. A significant p-value is indicated with exact value. In PRO-C18L tertile 1 (T1) the PL group had 16% regressors, 58% stable and 26% progressors while the T group had 13% regressors, 25% stable and 7% progressors. In PRO-C18L tertile 3 (T3) the PL group had 7% regressors, 60% stable and 33% progressors while T group har 22% regressors, 59% stable and 19% progressors.

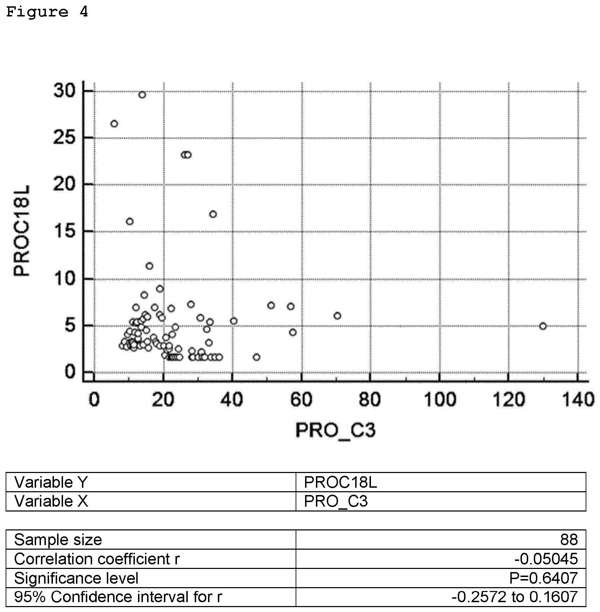

**Figure 4****:** Correlation coefficient of high PRO-C18L (above median of 2.56 ng/ml) and high PRO-C3

### Examples

The presently disclosed embodiments are described in the following Examples, which are set forth to aid in the understanding of the disclosure, and should not be construed to limit in any way the scope of the disclosure as defined in the claims which follow thereafter. The following examples are put forth so as to provide those of ordinary skill in the art with a complete disclosure and description of how to make and use the described embodiments, and are not intended to limit the scope of the present disclosure nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

In the following examples, the following materials and methods were employed.

### Materials and Methods

### Reagents

All reagents used for the experiment were high-quality chemicals from Merck (Whitehouse Station, NJ, USA) and Sigma Aldrich (St. Louis, MO, USA) and all synthetic peptides purchased from GenScript (Piscataway, NJ, USA). Sequences defining the peptides for antibody production and validation were as follows:
1. Standard peptide, NLLNLNKDKE (SEQ ID NO. 2), used for antibody production. This peptide differs in its last four amino acids as compared to the 10 amino acid N-terminus sequence of the long and intermediate isoforms of type XVIII collagen. The 10 amino acid N-terminus amino acid sequence of the long and intermediate isoforms of type XVIII collagen (formed after cleavage of the 23 amino acid signal peptide) is ²⁴NLLNLNWLWF³³ (SEQ ID NO. 14). This N-terminus sequence was found to be unique for human type XVIII collagen when tested for sequence homology against rat, mouse and bovine, but was also found to be insoluble and thus unsuitable for antibody production. Accordingly, the sequence NLLNLNKDKE (SEQ ID NO. 2), which is soluble, was used for antibody production instead, and specific binding of the produced antibodies to the long and intermediate isoforms of type XVIII collagen (and hence to the N terminus sequence NLLNLN (SEQ ID NO. 1)) was confirmed with subsequent testing. As used herein, the term "PRO-C18L" is used interchangeably to refer to both the standard peptide/sequence, NLLNLNKDKE (SEQ ID NO. 2), and to the N-terminus epitope/sequence, NLLNLN (SEQ ID NO. 1), that is present in both the standard peptide and the long and intermediate isoforms of type XVIII collagen and to which the antibodies described herein specifically bind.
2. Biotin conjugated peptide, NLLNLNKDKE-K-Biotin (SEQ ID NO. 15), for coating and screening
3. Immunogenic peptide, NLLNLNKDKEKK-GGC-KLH (SEQ ID NO. 16), conjugated with keyhole limpet hemocyanin (KLH) for immune reaction.

### Monoclonal antibody production

Six to seven weeks old Balb/C mice were immunized subcutaneously with 100 µg emulsified immunogen consisting of PRO-C18L keyhole limpet hemocyanin (KLH)-conjugated antigenic peptide (NLLNLNKDKEKK-GGC-KLH (SEQ ID NO. 16), the immunogenic peptide) mixed with Stimune Immunogenic Adjuvant (SPECOL, cat #7925000, Invitrogen). Immunization was repeated every second week until serum titer was found stable. The mouse with the highest serum titer and best peptide inhibition was selected for fusion. Thus, after resting at least three weeks, the selected mouse was given a final boost of 50-100 µg emulsified immunogen (depending on antibody titer) mixed with 100 µl 0.9% NaCl solution intraperitoneally. Three days following the final boost, the mouse were sacrificed and spleen cells isolated and fused with SP2/0 myeloma cells to generate hybridomas for further culturing, this procedure have previously been described¹⁰. Hybridomas were plated in 96-well microtiter plates, using semi-medium method and employing limited dilution to ensure monoclonality. In addition, the subcloning were repeated a minimum of two times to further ensure monoclonality. Supernatant was screened for reactivity and specificity against the standard peptide, a non-sense peptide (LHDSNPYPRR (SEQ ID NO. 17)) and a deselection peptide (NALNLNWLWF (SEQ ID NO. 18)) in a direct competitive ELISA using streptavidin-coated plates to confirm binding to the standard peptide and lack of binding to the non-sense peptide and deselection peptide. To confirm an IgG isotype the clone was further tested in a Clonotyping System-HRP kit, cat. No. 5300-05 (Southern Biotech, Birmingham, AL, USA).

The antibodies generated were sequenced and the CDRs determined.

The sequence of the chains are as follows:
CDRs underlined and in bold
Constant region italic:
   Heavy chain: Amino acid sequence (514 aa) 
QVQLQQPGAELVKPGASVKLSCKASGYTFIDYWIHWVKQRPGQGLEWIGEINPSDGRSNYNE
KFKTKATLTVDRSSSTAYMHLSSLTSEDSAVFYCARDLGFADWGPGTTVTVSSAKTTAPSVY
PLAPVCGDTTGSSVTLGCLVKGYFPEPVTLTWNSGSLSSGVHTFPAVLQSDLYTLSSSVTVT
SSTWPSQSITCNVAHPASSTKVDKKIEPRGPTIKPCPPCKCPAPNLLGGPSVFIFPPKIKDV
LMISLSPIVTCVVVDVSEDDPDVQISWFVNNVEVHTAQTQTHREDYNSTLRVVSALPIQHQD
WMSGKEFKCKVNNKDLPAPIERTISKPKGSVRAPQVYVLPPPEEEMTKKQVTLTCMVTDFMP
EDIYVEWTNNGKTELNYKNTEPVLDSDGSYFMYSKLRVEKKNWVERNSYSCSVVHEGLHNHH
TTKSFSRTPGLDLDDVCAEAQDGELDGLWTTITIFISLFLLSVCYSASVTLFKVKWIFSSVV
ELKQTISPDYRNMIGQGA (SEQ ID NO. 19)
   Light chain: Amino acid sequence (219 aa)

DILMTQTPLSLPVGLGDQASISCRSSQSIVYSNGNTYLQWYLQKPGQSPQLLIYKVSNRFSG
VPDRFSGSGSGTDFTLKISRVEAEDVGVYYCFQGSHVPFTFGSGTKLEIKRADAAPTVSIFP
PSSEQLTSGGASVVCFLNNFYPKDINVKWKIDGSERQNGVLNSWTDQDSKDSTYSMSSTLTL
TKDEYERHNSYTCEATHKTSTSPIVKSFNRNEC (SEQ ID NO. 20)

### Characterization of clones

Selected clones were purified using HiTrap affinity columns (GE Healthcare Life Science, Little Chalfont, Buckinghamshire, UK) and labelled with horseradish peroxidase (HRP) using Roche Peroxidase Labelling Kit, cat. no. 11829696001 (Roche Diagnostics GmbH, Mannheim, Germany) according to the manufacturer instructions. Native reactivity and affinity of the clones were assessed using different human biological material such as serum or plasma with EDTA, heparin or citrate.

### PRO-C18L competitive ELISA procedure

To measure the serological levels of PRO-C18L in serum or plasma samples, the following procedure is used:
1. Coat a 96-well streptavidin-coated microtiter plate cat. no. 11940279 from Roche Diagnostics GmbH (Mannheim, Germany) with 100 µl biotinylated peptide (the biotin conjugated peptide) dissolved in 50 mM PBS-BTB with 10% sorbitol and diluted in assay buffer (50 mM PBS-BTB, 8g NaCl, pH 7.4). Incubate in 20°C darkness on a shaking device of 300 rpm for 30 minutes.
2. Wash 5 times in washing buffer (20 mM Tris, 50 mM NaCl, pH 7.2).
3. Add 20 µl of the standard peptide, control or sample in duplicates to appropriate wells, followed by 100 µl HRP-labelled antibody diluted in assay buffer (50 mM PBS-BTB, 8g NaCl, pH 7.4). Incubate overnight in 4°C and shaking 300 rpm.
4. Wash 5 times in washing buffer (20 mM Tris, 50 mM NaCl, pH 7.2).
5. Add 100 µl tetramethylbenzinidine (TMB) (cat. no. 4380H, Kem-En-Tec, Taastrup, Denmark) and incubate for 15 minutes at 20°C.
6. Finally, add stopping solution (1% H2SO4)
7. Read the absorbance at 450 nm with 650 nm as reference. This is done on an ELISA microplate reader (VerseMax, Molecular Devices, Sunnyvale, CA, USA).

The standard curve is made by a 2-fold dilution of the standard peptide.

### Technical validation

To determine linearity of the measurements, recovery percentage of the 100% sample were determined in 2-fold dilutions of serum and plasma samples from human. Likewise, antibody specificity was calculated as recovery percentage of the 100% calibrator peptide (the standard peptide, NLLNLNKDKE (SEQ ID NO. 2)), the nonsense peptide (LHDSNPYPRR (SEQ ID NO. 17)) and the deselection peptide (NALNLNWLWF (SEQ ID NO. 18)). Lower limit of detection (LLOD) was determined as the mean +3x standard deviations (SD) of 21 blanks (buffer). Upper limit of detection (ULOD) was determined as the mean -3x SD of 10 measurements of Standard A. To assess the intra- and inter-assay variations, 10 independent runs of 8 quality control (QC) samples were measured in doublet determinations and calculated as mean coefficient of variance (CV%). Accuracy of the measurements was evaluated by measuring a healthy human serum sample spiked with standard peptide and calculated as the percentage recovery of the theoretical collected amount of analyte. Interference was measured as the percentage recovery of human serum sample spiked with haemoglobin, lipemia and biotin at significant concentrations. Acceptable range for linearity, accuracy and interference were ±20%.

### Analyte and reagent stability

Three serum and plasma human samples were subjected to five freeze-thaw cycles to determine stability of the analyte, with the first cycle serving as reference. In addition, the analyte stability was tested by incubating three serum and three plasma samples at 4°C and 20°C for 2-, 4-, 24- and 48-hours and tested against the non-stressed sample reference. All sample teste was run as doublet determinations. Reagent stability was tested by incubating the HRP labelled antibody at 20°C and 37°C for seven days and tested against a none-stressed reagent. Acceptable range for analyte and reagent stability tests were ±20%.

### Clinical evaluation

### Patient samples

PRO-C18L ELISA was tested in a cohort from a multicentre, phase II clinical study investigating the effectiveness of farglitazar, a peroxisome proliferator-activated receptor-gamma agonist, a potential antifibrotic compound for hepatitis C affected patients (NCT00244751) as described previously¹¹.

Plasma samples were available in a subpopulation of 200 patients receiving either daily doses of farglitazar or matching placebo for 52 weeks. Liver biopsies were available at baseline and after 52 weeks and viewed by a central experienced histopathologist using Ishak modified histological activity index for grading and staging. The study was conducted in compliance with the Declaration of Helsinki and followed ethical guidelines for all countries involved. All patients provided written informed consent. Baseline patient characteristics are shown in table 1 below.

**Table 1: Patient baseline characteristics**

| | **All, n = 127** | **Treatment (T), n = 87** | **Placebo (P), n = 40** | ***p-value T vs P*** |
|---|---|---|---|---|
| Age, years (range) | 52 (48-55) | 52 (48-56) | 52 (47-54) | *0.84* |
| Females (% n) | 50 (39%) | 37 (42%) | 13 (32%) | *0.87* |
| BMI, mean (±SD) | 28.8 (±4.8) | 28.5 (±4.5) | 29.6 (±5.4) | *0.21* |
| Race, n (%) | | | | |
| *White* | 101 (80%) | 70 (80%) | 31 (78%) | *0.80* |
| *Black* | 8 (6%) | 6 (7%) | 2 (5%) | *0.67* |
| *Asian* | 16 (12%) | 9 (11%) | 7 (17%) | *0.35* |
| *Other* | 2 (2%) | 2 (2%) | 0 (0%) | *0.37* |
| HAI, mean (±SD) | 6.4 (±1.6) | 6.4 (±1.7) | 6.4 (±1.5) | *0.94* |
| Steatosis grade, mean (±SD) | 1.2 (±0.7) | 1.2 (±0.6) | 1.2 (±0.7) | *0.84* |
| Collagen, median (IQR) | 0.05 (0.03-0.1 1) | 0.05 (0.03-0.1) | 0.04 (0.02-0.07) | *0.42* |
| AST, median (IQR) | 53 (38-77) | 56 (39-79) | 51 (32-71) | *0.80* |

| | | | | |
|---|---|---|---|---|
| *HAI, histologic activity index. BMI, body mass index. AST, aspartate aminotransferase. SD, standard deviation. IQR, interquartile range.* | | | | |

The cohort were selected for having chronic hepatitis C and difficult to treat with previous antiviral therapies failing. The initial study concluded that there were no effects of farglitazar, as the proportion of subjects with a progression or regression of disease, based on Ishak scoring, were the same from baseline to end of study¹¹. However, in a later publication on the same cohort a marker of type III collagen formation was found to identify responders to treatment, showing that serological measurements of fibrosis provides a more dynamic quantification of the disease activity¹².

### Biomarker measurements

PRO-C18L was measured in duplicates in EDTA plasma from the patients described above. The ELISA and measurements were developed and measured at Nordic Bioscience (Herlev, Denmark), as described above.

### Statistical analysis

### Tests used: Chi Square, T-test, one-way ANOVA

Graphs and statistical analysis were performed using MedCalc version 19.3 (MedCalc Software Ltd, Ostend, Belgium) and GraphPad Prism version 8 (GraphPad Software, La Jolla, CA, USA). Data is shown as Tukey box plots. A p-value of p<0.05 is considered statistically significant.

### Results

### Clone selection and quality assurance

To select the best antibody from the produced clones: native reactivity, specificity and stability was evaluated. The antibody chosen for the ELISA development was named NBH133A1-2B4 and determined to be an IgG2a subtype. To ensure antibody specificity, its reactivity was tested against the standard peptide, a non-sense peptide (LHDSNPYPRR (SEQ ID NO. 17)) and a deselection peptide (NALNLNWLWF (SEQ ID NO. 18)) using the competitive ELISA procedure described above. The standard peptide inhibited the signal in a dose-dependent manner (figure 1), whereas there was no reactivity against the non-sense peptide (data not shown) or deselection peptide (figure 1). This data shows that the selected monoclonal antibody has high specificity towards the PRO-C18L N-terminus epitope.

### ELISA Technical specifications

Technical evaluation of the PRO-C18L ELISA was assessed through the different validation steps described in the methods section above, and the results are summarized below in table 2.

**Table 2: Technical validation of the PRO-C18L assay**

| **Technical validation step** | | **PRO-C18L** | |
|---|---|---|---|
| Lower limit of detection (ng/ml) | | 1.7 | |
| Upper limit of detection (ng/ml) | | 282.5 | |
| Calculated midpoint, IC50 (ng/ml) | | 11.7 | |
| Intra-assay variation | | 8% | |
| Inter-assay variation | | 11% | |
| Serum / plasma samples | | Undiluted (recommended) | |
| Dilution recovery in serum | | 104% | |
| Dilution recovery in plasma (EDTA) | | 120% | |
| Dilution recovery in plasma (heparin) | | 100% | |
| Spiking recovery of calibrator peptide in serum | | 109% | |
| Analyte recovery, 4 freeze/thaw cycles (serum / EDTA plasma) | | 94% / 105% | |
| Serum sample recovery after 48h at 4°C/20°C | | 85%/116% | |
| EDTA plasma sample recovery after 48h at 4°C/20°C | | 102% / 90% | |
| Heparin plasma sample recovery after 48h at 4°C/20°C | | 82% / 109% | |
| Antibody stability 7 days, 20°C/37°C | | 124%/113% | |

| Interference test | | serum | plasma |
|---|---|---|---|
| | Biotin recovery, low/high | 99%/92% | 110%/102% |
| | Lipemia recovery, low/high | 103%/100% | 104%/101% |
| | Haemoglobin recovery, low/high | 108%/107% | 121%/99% |

The IC50 was 11.7 ng/ml. Lower and upper limit of detection was 1.7 ng/ml and 282.5 ng/ml. Intra- and inter-assay variations were 8% and 11%, which is below the accepted criteria limits of 10% and 15%, respectively. Dilution recovery in both serum and plasma, from undiluted to 1:4 dilution, was found to be within the accepted criteria of 100±20%. In a spiking test of calibration peptide (the standard peptide) in serum the mean recovery was found to be 109%; the binding affinity for biological fragment versus synthetic standard was the same, confirming N-terminus binding and high assay specificity. After testing the analyte with four freeze/thaw cycles the analyte recovery was 94% and 105% in serum and EDTA plasma, respectively. In addition, storage of serum, heparin plasma and EDTA plasma at 4°C and 20°C for 48 hours did not affect the analyte recoveries of 85% and 116% for serum, 82% and 109% for heparin plasma and 102% and 90% for EDTA plasma. No interference was detected from either low or high levels of biotin, lipemia or haemoglobin in serum and plasma with recoveries ranging from 92% - 121%. These results all together argue that PRO-C18L is a technically robust assay.

### Clinical validation of PRO-C18L

To test the potential of PRO-C18L as a biomarker of fibrosis, a longitudinal study of 127 HCV patients treated with farglitazar or placebo were measured. Demographics as tested in table 1 showed no significant difference in distribution between the treated and placebo group.

First the change in PRO-C18L from baseline (BL) to week 52 (W52) was assessed in placebo versus farglitazar treated patients, finding that placebo increased while treated patients decreased in PRO-C18L levels (p=0.05) as shown in figure 2A**.** To further test if higher baseline levels (above median) of PRO-C18L were consistent with more treatment response, above and below median of PRO-C18L (2.56 ng/ml) were tested in a similar manner: delta PRO-C18L from BL to W52 in treated vs placebo patients. Although, above median levels of baseline PRO-C18L visually decreased more with treatment than the below median treated patients (figure 2B), indicating modulations to the mean, the result was not significant. However, these results indicate that PRO-C18L is modulated by therapy.

To further evaluate PRO-C18L as a biomarker of liver fibrosis, patients were divided into regressors, stable or progressors based on delta Ishak from BL to W52. Regressors and progressors were characterized as having a decrease or increase, respectively, of one or more Ishak scores from BL to W52 while stable had no change in Ishak. To assess the predictive value of PRO-C18L, the placebo and farglitazar treatment groups were further divided into tertiles based on PRO-C18L levels at baseline. In the group of patients in tertile 3 (the tertile consisting of the patients with the highest PRO-C18L baseline levels) the farglitazar treatment group showed a significant difference in the proportion of regressors and progressors when compared to the placebo group (figure 3), whereas this difference was not found in tertile 1 patient group (the tertile consisting of the patients with the lowest PRO-C18L baseline levels)., suggesting that patients with high PRO-C18L levels at baseline are more likely to respond to treatment.

Finally, to assess if PRO-C18L is different from PRO-C3 (found to predict responders to treatment in an earlier study of the same patients) the two markers were correlated on their preliminary cut off levels (20.2 ng/ml for PRO-C3 and 2.56 ng/ml for PRO-C18L) (Figure 4). No correlation was found, suggesting that PRO-C18L identifies additional responders to treatment in the same population.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used to mean 'including or consisting of'. No acknowledgement of any prior published document herein should be taken to be an admission or representation that the teaching thereof was common general knowledge in Australia or elsewhere at the date hereof.

### References

1. Asrani, S. K., Devarbhavi, H., Eaton, J. & Kamath, P. S. Burden of liver diseases in the world. Journal of Hepatology vol. 70 151-171 (2019).
2. Faye, C., Chautard, E., Olsen, B. R. & Ricard-Blum, S. The first draft of the endostatin interaction network. J. Biol. Chem. 284, 22041-22047 (2009).
3. Kalluri, R. Basement membranes: Structure, assembly and role in tumour angiogenesis. Nature Reviews Cancer vol. 3 422-433 (2003).
4. Kjeld, N. G., Hua, B., Karsdal, M. A., Sun, S. & Manon-Jensen, T. The endothelial specific isoform of type XVIII collagen correlates to annual bleeding rate in haemophilia patients. PLoS One 13, 1-12 (2018).
5. Seppinen, L. & Pihlajaniemi, T. The multiple functions of collagen XVIII in development and disease. Matrix Biol. 30, 83-92 (2011).
6. Karsdal, M. A. Biochemistry of collagens, laminins and elastin: structure, function and biomarkers. (Elsevier, 2019).
7. Quélard, D. et al. A cryptic frizzled module in cell structure collagen 18 inhibits Wnt/β-catenin signaling. PLoS One 3, (2008).
8. Hendaoui, I. et al. Inhibition of Wnt/β-Catenin signaling by a soluble Collagen-Derived frizzled domain interacting with Wnt3a and the receptors frizzled 1 and 8. PLoS One 7, (2012).
9. Aikio, M. et al. Specific collagen XVIII isoforms promote adipose tissue accrual via mechanisms determining adipocyte number and affect fat deposition. Proc. Natl. Acad. Sci. U. S. A. 111, (2014).
10. Gefter, M. L., Margulies, D. H. & Scharff, M. D. A simple method for polyethylene glycol-promoted hybridization of mouse myeloma cells. Somatic Cell Genet. 3, 231-236 (1977).
11. McHutchison, J. et al. Farglitazar Lacks Antifibrotic Activity in Patients With Chronic Hepatitis C Infection. Gastroenterology 138, (2010).
12. Nielsen, M. J. et al. Plasma Pro-C3 (N-terminal type III collagen propeptide) predicts fibrosis progression in patients with chronic hepatitis C. Liver Int. 35, 429-437 (2014).

## Claims

1. A method of immunoassay for detecting or monitoring liver fibrosis in a patient or for assessing, in a patient with liver fibrosis, the likelihood of the patient responding to treatment with an antifibrotic drug, the method of immunoassay comprising:
(i) contacting a patient sample with a monoclonal antibody that specifically binds to the long and/or intermediate isoforms of type XVIII collagen and that does not specifically bind to the short isoform of type XVIII collagen, wherein the monoclonal antibody specifically binds to the amino acid sequence NLLNLN (SEQ ID NO. 1); and
(ii) detecting and determining the amount of binding between said monoclonal antibody and peptides in the sample.

2. The method of claim 1, wherein the patient sample is from a patient with liver fibrosis and the method is for assessing the likelihood of the patient responding to treatment with an antifibrotic drug, the method further comprising:
(iii) correlating said amount of binding of said monoclonal antibody as determined in step (ii) with values associated with responders to treatment and/or values associated with non-responders and/or a predetermined cut-off value.

3. The method of claim 1, wherein the method is for monitoring liver fibrosis in a patient, the method further comprising:
(iii) correlating said amount of binding of said monoclonal antibody as determined in step (ii) with values obtained from said patient at a previous time point.

4. The method of claim 3, wherein the patient sample is from a patient undergoing treatment with an antifibrotic drug.

5. The method of any preceding claim, wherein the patient sample is from a patient with chronic liver disease.

6. The method of any preceding claim, wherein the patient sample is from a patient with viral hepatitis, alcoholic liver disease, or non-alcoholic fatty liver disease.

7. The method of claim 1, wherein the method is for detecting liver fibrosis in a patient, the method further comprising:
(iii) correlating said amount of binding of said monoclonal antibody as determined in step (ii) with values associated with normal healthy subjects and/or values associated with known disease severity and/or a predetermined cut-off value.

8. The method of any preceding claim, wherein the sample is a biofluid.

9. The method of claim 8, wherein the biofluid is blood, serum or plasma.

10. The method of any preceding claim, wherein said immunoassay is a competition assay or a sandwich assay.

11. The method of any preceding claim, wherein said immunoassay is a radioimmunoassay or an enzyme-linked immunosorbent assay.

12. The method of any preceding claim, wherein the monoclonal antibody is raised against a synthetic peptide having the amino acid sequence NLLNLNKDKE (SEQ ID NO. 2).

## Patentansprüche

1. Ein Immunoassay-Verfahren zum Nachweisen oder Überwachen von Leberfibrose bei einem Patienten oder zum Beurteilen der Wahrscheinlichkeit, dass ein Patient mit Leberfibrose auf eine Behandlung mit einem antifibrotischen Medikament anspricht, das Immunoassay-Verfahren umfassend:
(i) In-Kontakt-Bringen einer Patientenprobe mit einem monoklonalen Antikörper, der spezifisch an die langen und/oder intermediären Isoformen von Kollagen Typ XVIII bindet und der nicht spezifisch an die kurze Isoform von Kollagen Typ XVIII bindet, wobei der monoklonale Antikörper spezifisch an die Aminosäuresequenz NLLNLN (SEQ ID NR. 1) bindet; und
(ii) Nachweisen und Bestimmen des Ausmaßes der Bindung zwischen dem monoklonalen Antikörper und Peptiden in der Probe.

2. Verfahren nach Anspruch 1, wobei die Patientenprobe von einem Patienten mit Leberfibrose stammt und das Verfahren dazu dient, die Wahrscheinlichkeit zu beurteilen, dass der Patient auf eine Behandlung mit einem antifibrotischen Medikament anspricht, das Verfahren ferner umfassend:
(iii) Korrelieren der in Schritt (ii) bestimmten Bindungsmenge des monoklonalen Antikörpers mit Werten, die mit auf die Behandlung ansprechenden Patienten assoziiert sind, und/oder mit Werten, die mit nicht ansprechenden Patienten assoziiert sind, und/oder mit einem vorbestimmten Grenzwert.

3. Verfahren nach Anspruch 1, wobei das Verfahren zum Überwachen von Leberfibrose bei einem Patienten dient, das Verfahren ferner umfassend:
(iii) Korrelieren der in Schritt (ii) bestimmten Bindungsmenge des monoklonalen Antikörpers mit Werten, die von dem Patienten zu einem früheren Zeitpunkt erhalten wurden.

4. Verfahren nach Anspruch 3, wobei die Patientenprobe von einem Patienten stammt, der sich einer Behandlung mit einem antifibrotischen Medikament unterzieht.

5. Verfahren nach einem der vorhergehenden Ansprüche, wobei die Patientenprobe von einem Patienten mit chronischer Lebererkrankung stammt.

6. Verfahren nach einem der vorhergehenden Ansprüche, wobei die Patientenprobe von einem Patienten mit viraler Hepatitis, alkoholischer Lebererkrankung oder nichtalkoholischer Fettlebererkrankung stammt.

7. Verfahren nach Anspruch 1, wobei das Verfahren zum Nachweisen von Leberfibrose bei einem Patienten dient, das Verfahren ferner umfassend:
(iii) Korrelieren der in Schritt (ii) bestimmten Bindungsmenge des monoklonalen Antikörpers mit Werten, die mit normalen gesunden Probanden assoziiert sind, und/oder mit Werten, die mit einem bekannten Schweregrad der Erkrankung assoziiert sind, und/oder mit einem vorbestimmten Grenzwert.

8. Verfahren nach einem der vorhergehenden Ansprüche, wobei die Probe ein Biofluid ist.

9. Verfahren nach Anspruch 8, wobei das Biofluid Blut, Serum oder Plasma ist.

10. Verfahren nach einem der vorhergehenden Ansprüche, wobei der Immunassay ein Kompetitionsassay oder ein Sandwich-Assay ist.

11. Verfahren nach einem der vorhergehenden Ansprüche, wobei der Immunoassay ein Radioimmunoassay oder ein enzymgekoppelter Immunadsorptionsassay ist.

12. Verfahren nach einem der vorhergehenden Ansprüche, wobei der monoklonale Antikörper gegen ein synthetisches Peptid mit der Aminosäuresequenz NLLNLNKDKE (SEQ ID NR. 2) gerichtet ist.

## Revendications

1. Procédé de dosage immunologique pour détecter ou surveiller une fibrose hépatique chez un patient ou pour évaluer, chez un patient atteint de fibrose hépatique, la probabilité que le patient réponde au traitement par un médicament antifibrotique, le procédé de dosage immunologique comprenant les étapes consistant à :
(i) mettre un échantillon provenant d'un patient en contact avec un anticorps monoclonal qui se lie spécifiquement à l'isoforme longue et/ou à l'isoforme intermédiaire du collagène de type XVIII et qui ne se lie pas spécifiquement à l'isoforme courte du collagène de type XVIII, l'anticorps monoclonal se liant spécifiquement à la séquence d'acides aminés NLLNLN (SEQ ID NO. 1) ; et
(ii) détecter et déterminer la quantité de liaison entre ledit anticorps monoclonal et des peptides dans l'échantillon.

2. Procédé selon la revendication 1, dans lequel l'échantillon provenant d'un patient provient d'un patient atteint de fibrose hépatique et le procédé est pour évaluer la probabilité que le patient réponde au traitement par un médicament antifibrotique, le procédé comprenant en outre :
(iii) corréler ladite quantité de liaison dudit anticorps monoclonal, telle que déterminée dans l'étape (ii), avec des valeurs associées à des patients répondant au traitement et/ou des valeurs associées à des patients non répondeurs et/ou une valeur seuil préétablie.

3. Procédé selon la revendication 1, le procédé étant pour surveiller une fibrose hépatique chez un patient, le procédé comprenant en outre :
(iii) corréler ladite quantité de liaison dudit anticorps monoclonal telle que déterminée dans l'étape (ii) avec des valeurs obtenues dudit patient à un moment précédent.

4. Procédé selon la revendication 3, dans lequel l'échantillon provenant d'un patient provient d'in patient soumis à un traitement par un médicament antifibrotique.

5. Procédé selon l'une quelconque des revendications précédentes, dans lequel l'échantillon provenant d'un patient provient d'un patient atteint d'hépatopathie chronique.

6. Procédé selon l'une quelconque des revendications précédentes, dans lequel l'échantillon provenant d'un patient provient d'un patient atteint d'hépatite virale, d'hépatopathie alcoolique, ou de stéatose hépatique non alcoolique.

7. Procédé selon la revendication 1, le procédé étant pour détecter une fibrose hépatique chez un patient, le procédé comprenant en outre :
(iii) corréler ladite quantité de liaison dudit anticorps monoclonal telle que déterminée dans l'étape (ii) avec des valeurs associées à des sujets sains normaux et/ou des valeurs associées à une gravité connue de maladie et/ou une valeur seuil préétablie.

8. Procédé selon l'une quelconque des revendications précédentes, dans lequel l'échantillon est un liquide biologique.

9. Procédé selon la revendication 8, dans lequel le liquide biologique est le sang, le sérum ou le plasma.

10. Procédé selon l'une quelconque des revendications précédentes, dans lequel ledit dosage immunologique est un dosage compétitif ou un dosage de type sandwich.

11. Procédé selon l'une quelconque des revendications précédentes, dans lequel ledit dosage immunologique est un dosage radio-immunologique ou un dosage par immunoadsorption à enzyme liée.

12. Procédé selon l'une quelconque des revendications précédentes, dans lequel l'anticorps monoclonal est suscité contre un peptide synthétique ayant la séquence d'acides aminés NLLNLNKDKE (SEQ ID NO. 2.
